Europäisches Patentamt

European Patent Office

Office européen des brevets

(19)

(11) Veröffentlichungsnummer : **0 092 541**
**B1**

(12) **EUROPÄISCHE PATENTSCHRIFT**

(45) Veröffentlichungstag der Patentschrift :
11.02.87

(51) Int. Cl.⁴ : **A 61 B   5/10**, G 01 B   9/10

(21) Anmeldenummer : 83890055.3

(22) Anmeldetag : 15.04.83

(54) Vorrichtung zur Diagnostizierung von Störungen des Bewegungssystems des menschlichen Körpers.

Verbunden mit 83901175.6/0120016 (europäische Anmeldenummer/Veröffentlichungsnummer) durch Entscheidung vom 20.03.85.

(30) Priorität : 16.04.82 AT 1485/82

(43) Veröffentlichungstag der Anmeldung :
26.10.83 Patentblatt 83/43

(45) Bekanntmachung des Hinweises auf die Patenterteilung : 11.02.87 Patentblatt 87/07

(84) Benannte Vertragsstaaten :
AT BE CH DE FR GB IT LI NL SE

(56) Entgegenhaltungen :
DE-A- 2 912 981
US-A- 3 161 846
US-A- 3 290 985
US-A- 3 664 731
US-A- 4 306 571
Die Akte enthält technische Angaben, die nach dem Eingang der Anmeldung eingereicht wurden und die nicht in dieser Patentschrift enthalten sind.

(73) Patentinhaber : Berger, Meinhard, Dr.(med.)
Nr. 55
A-6103 Reith (AT)

(72) Erfinder : Berger, Meinhard, Dr.(med.)
Nr. 55
A-6103 Reith (AT)

(74) Vertreter : Hofinger, Engelbert et al
Torggler-Hofinger Wilhelm-Greil-Strasse 16
A-6020 Innsbruck (AT)

## Beschreibung

Die Erfindung bezieht sich auf eine Vorrichtung zur Diagnostizierung von Störungen des Bewegungssystems des menschlichen Körpers durch Messung der Stellung und/oder Bewegung eines Körperteiles, wobei zumindest seine Rotationen mit Hilfe einer Meßvorrichtung gemessen werden, die aus einem mit dem Körperteil fest zu verbindenden Halteteil, einer raumfest montierten Trägervorrichtung für den Halteteil und einem aus Meßfühlern zur Erfassung der Rotationsbewegungen des Halteteiles in bezug auf die Trägervorrichtung bestehenden Meßsystems besteht.

Für Untersuchungen, insbesondere in den Fachgebieten der Orthopädie und der Neuro-Orthopädie, die sich mit funktionellen Störungen des Bewegungssystems beschäftigt, ist es wichtig, gewisse Stellungen und Bewegungen von Körperteilen meßtechnisch zu erfassen und diagnostisch auszuwerten. Beispielsweise sind durch die Registrierung der Stellung und Bewegung des Kopfes Rückschlüsse auf die Funktion der Halswirbelsäule und der oberen Brustwirbelsäule möglich. Funktionsstörungen dieser Wirbelsäulenabschnitte sind die häufigste Ursache der Beschwerden von Oberkörper, Armen und Kopf. Mittels der derzeit gebräuchlichen Untersuchungsverfahren (Röntgen, Scan etc.) sind wohl statische Untersuchungen möglich, die Untersuchung der Dynamik und Funktion der Wirbelsäule aber nur sehr eingeschränkt durchführbar. Es setzt sowohl die Technik dieses Verfahrens als auch die Strahlenbelastung enge Grenzen für Funktionsuntersuchungen der Wirbelsäule. Durch optische Kontrollverfahren (Film, Video) ist eine dreidimensionale Registrierung der Lage des Kopfes nur mit großem apparativem Aufwand und für Einzelfunktionen möglich.

Zur Messung von Kopfbewegungen ist auch bereits eine Vorrichtung der eingangs genannten Gattung bekannt geworden (US-A-3,161,846, US-A-3,290,985), die sich aber für diagnostische Zwecke nicht eignet, weil die freie Beweglichkeit des Kopfes zu stark eingeschränkt ist, und somit z. B. die diagnostisch wesentlichen Endlagen der Kopfbewegungen und rasche Bewegungen nicht erfaßt werden könnten. Weiters läßt die bekannte Vorrichtung einen Zugriff des Arztes zum Kopf nicht in dem für eine diagnostische Anwendung erforderlichen Maße zu.

Aufgabe der Erfindung ist es daher, eine Vorrichtung zur Messung der Stellung und/oder Bewegung des Kopfes bzw. anderer Körperteile der eingangs genannten Gattung zu schaffen, welche den diagnostischen Erfordernissen entspricht.

Dies wird erfindungsgemäß dadurch erreicht, daß der Halteteil mit der Trägervorrichtung an einer einzigen Stelle über eine eine freie Bewegung des Körperteiles zulassende gelenkige Verbindung verbunden ist, und daß die Trägervorrichtung so ausgebildet ist, daß Bewegungen des Meßsystems bzw. des Halteteiles in einem raumfesten Koordinatensystem erfaßbar sind.

Der Erfindung liegt speziell für die Funktionsuntersuchung der Halswirbelsäule und der oberen Brustwirbelsäule die Erkenntnis zugrunde, daß allein schon aus der Messung der Rotation des Kopfes die wesentlichen diagnostischen Aussagen abgeleitet werden können. Aber auch in anderen Fällen können Rotationsbewegungen von Körperteilen, z. B. des Oberarmes, der Hand, des Fußes usw., in einem raumorientierten Koordinatensystem diagnostisch verwertbare Aussagen vermitteln. Den diagnostischen Erfordernissen entspricht die erfindungsgemäße Vorrichtung insbesondere dadurch, daß eine möglichst freie Beweglichkeit des zu messenden Körperteiles bei geringer Massenträgheit gewährleistet ist, um so auch rasche Bewegungen sowie die Endlagen der Bewegungen des Körperteiles erfassen zu können und auch solche Rückwirkungen der Meßvorrichtung auf die Körperteilbewegung zu vermeiden, die reflektorisch hervorgerufene Änderungen der Bewegung bewirken und somit die diagnostischen Aussagen der Messung verfälschen könnten. Außerdem wird dadurch, daß sich die Verbindung des Halteteiles mit der Trägervorrichtung praktisch auf einen Punkt (nämlich jenen der einen gelenkigen Verbindung) konzentriert, der gegebenenfalls während des Meßvorganges erforderliche Zugriff des Arztes zum Körperteil gewährleistet.

Eine bevorzugte Ausführungsform, die eine einfache konstruktive Lösung ermöglicht, besteht darin, daß die gelenkige Verbindung zwischen Halteteil und Trägervorrichtung durch das Meßsystem selbst erfolgt, welches vorzugsweise aus mindestens drei eine kinematische Kette bildenden Rotationsmeßfühlern besteht, deren Rotationsachsen ein dreidimensionales Achssystem definieren.

Die kinematische Kette der Rotationsmeßfühler kann dabei zweckmäßig so ausgebildet sein, daß die Rotationsmeßfühler in Serie angeordnet sind, wobei die relativ zueinander beweglichen Glieder der Rotationsmeßfühler derart angeordnet sind, daß das eine Glied des ersten Rotationsmeßfühlers mit dem Halteteil, das andere Glied des ersten Rotationsmeßfühlers mit einem Glied des zweiten Rotationsmeßfühlers verbunden ist, und daß ein Glied des letzten Rotationsmeßfühlers mit einem Teil der Trägervorrichtung verbunden ist.

Als Meßfühler können im Rahmen der erfindungsgemäßen Vorrichtung sogenannte Drehgeber verwendet werden. Drehgeber dienen zum Umformen einer als Drehbewegung vorliegenden Meßgröße in eine zur elektrischen Verarbeitung geeignete Größe, wie Widerstand, Strom, Spannung od. dgl. Als Beispiel eines geeigneten Drehgebers sei das drehbare Potentiometer (ein Widerstands-Drehgeber) genannt.

Anstelle von drehbaren Potentiometern können als Meßfühler auch andere Drehgeber (z. B. induktive oder kapazitive) bzw. auch digitale Winkelkodierer (optisch, magnetisch) verwendet werden.

Weitere besondere Ausführungen der Erfindung sind in den abhängigen Ansprüchen angegeben.

Die Erfindung wird nachstehend anhand der Zeichnungen durch Ausführungsbeispiele näher erläutert.

Figur 1 zeigt eine Seitenansicht und

Figur 2 eine Vorderansicht einer Meßvorrichtung für die Messung der Bewegung des Kopfes.

Figur 3 ist ein Schnitt gemäß III-III der Fig. 1.

Figur 4 zeigt eine Gesamtansicht der Meßvorrichtung mit weiteren Einzelheiten der Trägervorrichtung.

Figur 5 zeigt eine Ausführungsvariante zu Fig. 1 in Seitenansicht.

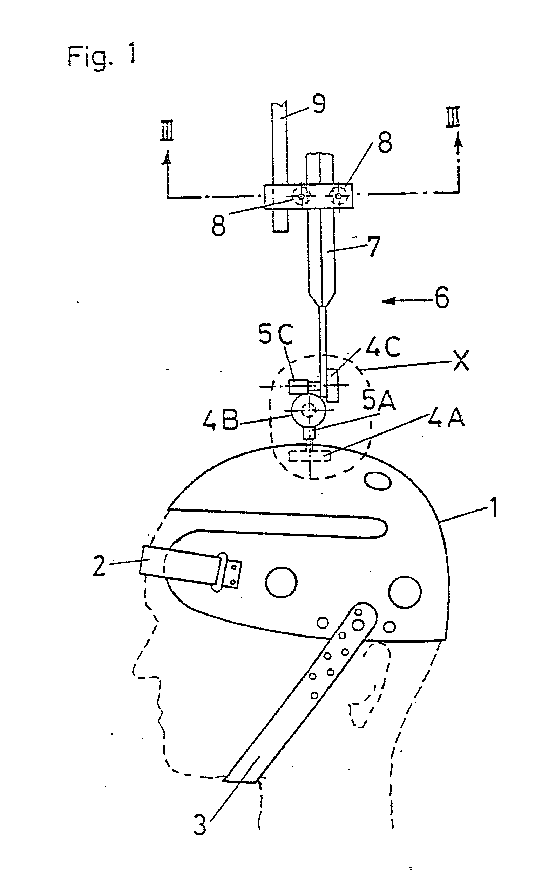

Das Ausführungsbeispiel nach Fig. 1 bis 4 zeigt eine Vorrichtung zur Messung der Stellung und Bewegung des Kopfes um drei Achsen zur Funktionsuntersuchung der Halswirbelsäule, der oberen Brustwirbelsäule und funktionell zugeordneten Strukturen.

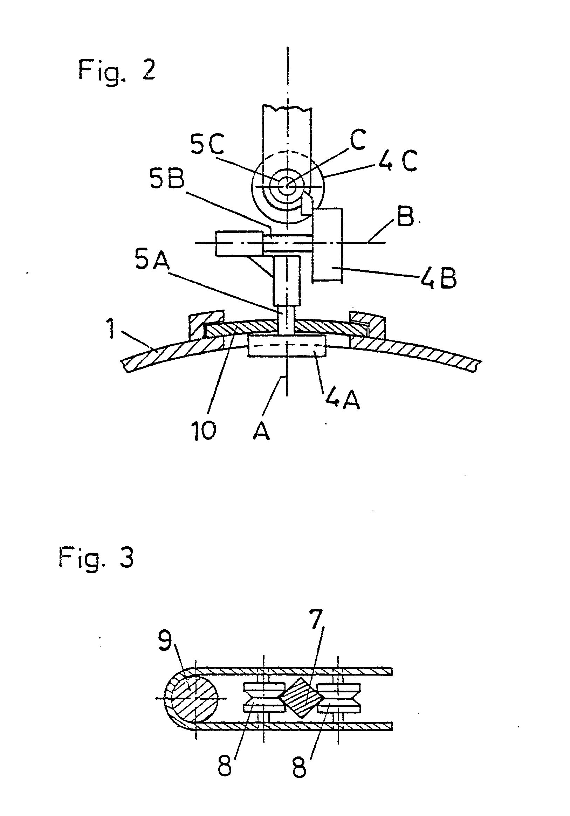

Der mit dem Körperteil zu verbindende Halteteil besteht im Falle des dargestellten Ausführungsbeispiels aus einem Helm 1 aus Kunststoff, der mittels verstellbarem Stirnband 2 und Kinnriemen 3 den verschiedenen Kopfgrößen und Kopfformen angepaßt werden kann. An der Oberseite des Helmes befindet sich als Meßsystem X eine Anordnung von drei drehbaren Potentiometern, deren Achsen A, B, C in der in Fig. 1 und 2 dargestellten Ausgangsposition (Nullstellung) jeweils um 90° versetzt sind. Das Gehäuse 4A des untersten Potentiometers ist am Helm 1 befestigt, seine Welle 5A liegt in der A-Achse. Die Welle 5B des zweiten Potentiometers ist mit der Welle 5A des ersten Potentiometers starr verbunden und liegt in der B-Achse. Das Gehäuse 4B des zweiten Potentiometers ist mit der Welle 5C des dritten . Potentiometers starr verbunden, die in der C-Achse liegt. Das Gehäuse 4C des dritten Potentiometers ist mit der Trägervorrichtung 6 fest verbunden. Das Meßsystem X bildet gleichzeitig auch die einzige gelenkige Verbindung zwischen Halteteil (Helm 1) und Trägervorrichtung 6.

Zur Justierung des ersten Potentiometers 4A-5A gegenüber dem Helm 1 ist in einer Ausnehmung des Helmes 1 eine Justierplatte 10 vorgesehen, an der das Potentiometergehäuse 4A unmittelbar befestigt und die in der sagittalen Ebene relativ zum Helm 1 verstellbar ist.

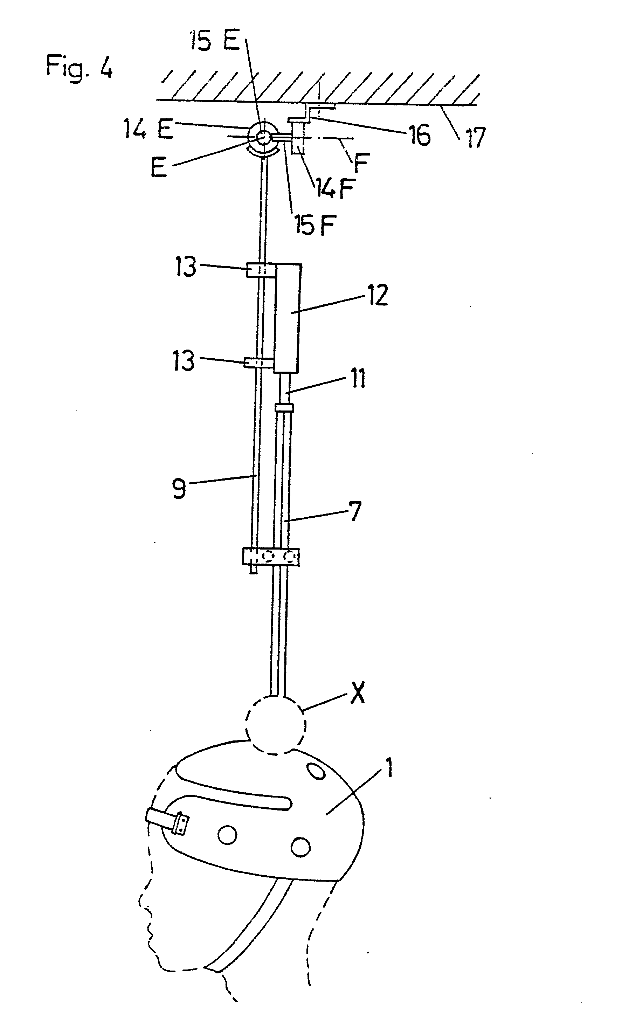

Die Trägervorrichtung 6 besteht aus einer Trägerstange 7 aus Leichtmetall mit rechteckigem Profil. Die Trägerstange 7 ist gewichtsentlastet der Höhe nach frei beweglich, jedoch in bezug auf ihre vertikale Achse drehfest angeordnet. Die Trägerstange 7 ist mit Hilfe von Laufrollen 8 an einer Führungsstange 9 geführt. Die Führungsstange 9 ist mittels eines Gelenkes an der Decke des Untersuchungsraumes fixiert, derart, daß kleine Pendelbewegungen des Führungsstange 9 und damit der Trägerstange 7 möglich sind, nicht aber Drehbewegungen der Trägerstange 7 um deren (hier etwa vertikalen) Längsachse. Wie aus Fig. 4 ersichtlich, kann auch die Längsverschiebung der Trägerstange 7 gegenüber der Führungsstange 9 gemessen werden, z. B. durch ein Linearpotentiometer 11, 12, dessen beweglicher Funktionsteil 11 mit der Trägerstange 7 und dessen Gehäuse 12 mit Hilfe von Laschen 13 an der Führungsstange 9 befestigt ist. Gemessen werden kann ferner der Winkelausschlag der Führungsstange 9, und zwar gemäß Fig. 4 durch zwei Potentiometer 14E-15E und 14F-15F, die gleichzeitig auch die gelenkige Verbindung zwischen Führungsstange und Decke 17 darstellen. Das Gehäuse 14E des einen Potentiometers ist mit dem Ende der Führungsstange 9 verbunden. Die Welle 15E dieses Potentiometers und die Welle 15F des anderen Potentiometers stehen in starrer Verbindung. Das Gehäuse 14F des anderen Potentiometers ist über ein Winkelstück 16 mit der Decke 17 verbunden. Die gelenkige Verbindung der Führungsstange 9 mit der Decke 17 gestattet Ausschläge der Führungsstange 9 um die Achsen E und F.

Durch die drei Potentiometer des Meßsystems X werden die Meßgrößen (Drehwinkel) der Kopfbewegungen um die drei Achsen A, B, C in einen elektrischen Widerstand als Ausgangsgröße umgeformt (Widerstands-Drehgeber). Die Ausgangsgröße jeder der drei Potentiometer werden als elektrische Signale einem Anzeigegerät (z. B. Oszilloskop), Aufzeichnungsgerät (Kurvenschreiber) und/oder Datenverarbeitungsgerät zugeführt, wobei die von den einzelnen Potentiometern kommenden Meßsignale sowohl gesondert als auch in Kombination, d. h. in Überlagerungen angezeigt, aufgezeichnet oder EDV-verarbeitet werden können. Dem Anzeige-Aufzeichnungs- und/oder Datenverarbeitungsgerät können auch die Ausgangsgrößen der zusätzlichen Meßfühler in bzw. an der Trägervorrichtung 6 entweder zur gesonderten Anzeige oder zur Verarbeitung als Korrekturgrößen der Rotationsmessungen zugeführt werden. Damit können mit der Meßvorrichtung auch die Transversalbewegungen des Halteteiles (Helmes 1) exakt erfaßt werden, die sich zwangsläufig mit den Rotationsbewegungen als Begleitbewegungen abspielen.

Mit der beschriebenen und in Fig. 1 bis 4 dargestellten Meßvorrichtung wird wie folgt verfahren:

Der Patient sitzt angelehnt im Stuhl und hält den Kopf gerade. Der Helm 1 wird so am Kopf montiert, daß die drei Potentiometer in der Nullstellung mit ihren Achsen A, B, C ein dreidimensionales Achssystem mit zueinander um 90° versetzten Achsen bilden. Die A-Achse wird längs der Körperlängsachse senkrecht ausgerichtet. Die B-Achse befindet sich in der Nullstellung horizontal · und sagittal, die C-Achse ebenfalls horizontal, aber frontal (parallel Ohr-Ohr). Durch die beschriebene Meßvorrichtung wird die Rotation, Flexion und Seitenneigung des Kopfes erfaßt, wobei während des Meßvorganges die A-Achse körperteilorientiert (kopforientiert) und die C-Achse raumorientiert bleibt. Die B-Achse bleibt bei der bloßen Rotation des Kopfes um die A-Achse (zusammen mit der C-Achse) raumorientiert, bei der bloßen Seitenneigung des Kopfes (zusammen mit der A-Achse) jedoch körperteilorientiert.

Raumorientiert bedeutet nicht unbedingt raumfest, denn die raumorientierte C-Achse des dritten Potentiometers kann infolge der gelenkigen Aufhängung der Führungsstange 9 und der im wesentlichen vertikalen Beweglichkeit der Trägerstange 8 den Kopfbewegungen entsprechende Begleitbewegungen ausführen, wobei jedoch die C-Achse stets in der der Sagittalebene der Nullstellung entsprechenden Vertikalebene bleibt. Die relativ geringen Begleitbewegungen der C-Achse wirken sich auf das Meßergebnis nicht wesentlich aus und könnten zudem in einem Datenverarbeitungsgerät kompensiert werden, insbesondere dann, wenn die Ausschläge und die Längenveränderungen der Trägervorrichtungen z. B., wie aus Fig. 4 ersichtlich, durch die Potentiometer 11-12, 14E-15E, 14F-15F oder andere Meßfühler gemessen werden.

Auf Grund des mit Hilfe der beschriebenen Vorrichtung durchgeführten Meßverfahrens kann einmal durch endlagige Rotation, Flexion und Extension, sowie Seitenneigung des Kopfes, das Bewegungsausmaß der oberen Halswirbelsäule und Brustwirbelsäule registriert werden. Durch langsame und rasche Hin- und Her-Rotation bzw. Flexion und Seitenneigung kann ferner der dynamische Ablauf dieser Bewegungen exakt registriert werden. Da wegen der Gelenksmechanik der oberen Wirbelsäulenabschnitte keine Bewegungen in nur einer Ebene möglich sind, das heißt, nur mehrdimensionale Bewegungen ablaufen können, ist die Registrierung der Synkinesen (Kombination von Rotation, Seitenneigung und Flexion) für die Diagnose von Störungen der Wirbelsäule äußerst wichtig. Durch Messung der Winkelgeschwindigkeit und der Frequenz von wiederholten Bewegungen lassen sich Rückschlüsse auf Muskeltonus, Verletzungshemmung, sowie nervale Steuerungsstörungen der Muskulatur (z. B. bei Parkinson, Zustand nach Lähmungen, etc.) durchführen. Durch Fixation einzelner Wirbel der Halswirbelsäule kann die Beweglichkeit einzelner Halswirbelsäulenabschnitte selektiv geprüft und registriert werden.

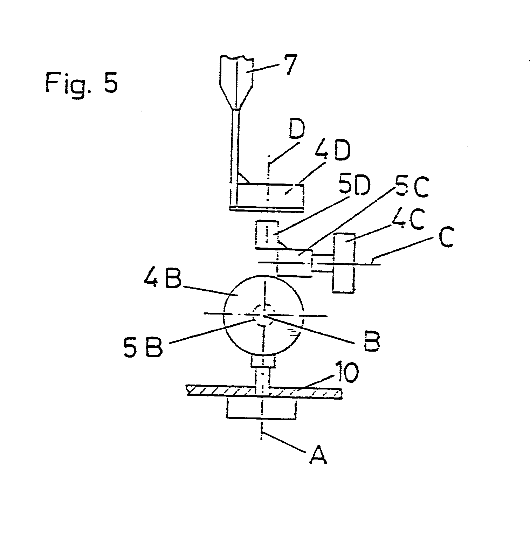

Als Ausführungsvariante zur Meßvorrichtung gemäß Fig. 1 bis 4 sei die Möglichkeit des Einbaues eines vierten Potentiometers erwähnt. Dieses könnte z. B. — wie aus Fig. 5 ersichtlich — zwischen dem dritten Potentiometer 4C-5C und der Trägerstange 7 angeordnet sein, derart, daß die Achse D des vierten Potentiometers in der im wesentlichen vertikalen Achse der Trägerstange 7 liegt, und das Gehäuse 4D des vierten Potentiometers mit der Trägerstange 7 fest verbunden wird, während die Welle 5D des vierten Potentiometers mit der Welle 5C des dritten Potentiometers in starrer Verbindung steht. In der Nullstellung fallen die Achsen D und A des vierten und ersten Potentiometers zusammen.

Für die Messung muß entweder das erste oder vierte Potentiometer gesperrt (d. h. die Welle gegenüber dem Gehäuse fixiert) werden. Bei gesperrtem ersten Potentiometer bleibt während der Messung die vertikale D-Achse raumorientiert, während die horizontale B-Achse körperteilorientiert (kopforientiert) bleibt.

Eine mögliche Ausführungsvariante besteht gegenüber einer Potentiometeranordnung nach Fig. 5 darin, das unterste Potentiometer wegzulassen und statt dessen das Potentiometer 4B-5B mit dem Helm bzw. der Justierplatte 10 zu verbinden.

Obgleich das Wesen der erfindungsgemäßen Vorrichtung die Messung von Drehbewegungen von Körperteilen betrifft, kann selbstverständlich in Verbindung damit auch die Messung von Transversalbewegungen der Körperteile erfolgen, wobei die Meßwerte der Drehbewegungen und der Transversalbewegungen entweder gesondert oder kombiniert mit den Meßwerten der Drehbewegungen zur Anzeige, Aufzeichnung oder EDV-Verarbeitung gebracht werden.

## Patentansprüche

1. Vorrichtung zur Diagnostizierung von Störungen des Bewegungssystems des menschlichen Körpers durch Messung der Stellung und/oder Bewegung eines Körperteiles, wobei zumindest seine Rotationen mit Hilfe einer Meßvorrichtung gemessen werden, die aus einem mit dem Körperteil fest zu verbindenden Halteteil (1), einer raumfest montierten Trägervorrichtung (6) für den Halteteil (1) und einem aus Meßfühlern zur Erfassung der Rotationsbewegungen des Halteteiles in bezug auf die Trägervorrichtung bestehenden Meßsystems (X) besteht, dadurch gekennzeichnet, daß der Halteteil (1) mit der Trägervorrichtung (6) an einer einzigen Stelle über eine eine freie Bewegung des Körperteiles zulassende gelenkige Verbindung verbunden ist, und daß die Trägervorrichtung (6) so ausgebildet ist, daß Bewegungen des Meßsystems (X) bzw. des Halteteiles (1) in einem raumfesten Koordinatensystem erfaßbar sind.

2. Vorrichtung nach Anspruch 1, dadurch gekennzeichnet, daß die gelenkige Verbindung zwischen Halteteil (1) und Trägervorrichtung (6) durch das Meßsystem (X) selbst erfolgt, welches vorzugsweise aus mindestens drei eine kinematische Kette bildenden Rotationsmeßfühlern (4A bis D, 5A bis D) besteht, deren Rotationsachsen (A, B, C ; B, C, D) ein dreidimensionales Achssystem definieren.

3. Vorrichtung nach Anspruch 2, dadurch gekennzeichnet, daß die Rotationsmeßfühler (4A bis D, 5A bis D) in an sich bekannter Weise Drehgeber, beispielsweise drehbare Potentiometer sind.

4. Vorrichtung nach Anspruch 2 oder 3, dadurch gekennzeichnet, daß die Rotationsmeßfühler (4A bis D, 5A bis D) in Serie angeordnet sind, wobei die relativ zueinander beweglichen Glieder der Rotationsmeßfühler derart angeordnet sind, daß das eine Glied (4A) des ersten Rotationsmeßfühlers mit dem Halteteil (1), das andere Glied (5A) des ersten Rotationsmeßfühlers mit einem Glied (z. B. 5B) des zweiten Rotationsmeßfühlers verbunden ist, und daß ein Glied (4C, 4D) des letzten Rotationsmeßfühlers mit einem Teil (7) der Trägervorrichtung (6) verbunden ist.

5. Vorrichtung nach Anspruch 4, wobei insbesondere zur Messung der Stellung und Bewegung des Kopfes drei Drehgeber, z. B. drehbare Potentiometer vorgesehen sind, dadurch gekennzeichnet, daß das Gehäuse (4A) des ersten Drehgebers mit dem Körperteil, z. B. mittels eines Helmes (1) mit dem Kopf, die Welle (5A) des ersten Drehgebers mit der Welle (5B) des zweiten Drehgebers und das Gehäuse (4B) des zweiten Drehgebers mit der Welle (5C) des dritten Drehgebers verbunden ist, dessen Gehäuse (4C) an einem Teil (7) der raumfest montierten Trägervorrichtung (6) befestigt ist.

6. Vorrichtung nach Anspruch 4, wobei insbesondere zur Messung der Stellung und Bewegung des Kopfes drei Drehgeber, z. B. drehbare Potentiometer vorgesehen sind, dadurch gekennzeichnet, daß die Welle des ersten Drehgebers mit dem Körperteil, z. B. mittels eines Helmes mit dem Kopf, das Gehäuse des ersten Drehgebers mit der Welle des zweiten Drehgebers und dessen Gehäuse mit der Welle des dritten Drehgebers verbunden ist, dessen Gehäuse an einem Teil der raumfest montierten Trägervorrichtung befestigt ist.

7. Vorrichtung nach Anspruch 5, dadurch gekennzeichnet, daß zwischen dem dritten Drehgeber und der Trägervorrichtung (6) ein vierter Drehgeber (4D, 5D) angeordnet ist, dessen Welle (5D) mit der Welle (5C) des dritten Drehgebers und dessen Gehäuse (4D) mit einem Teil (7) der Trägervorrichtung (6) verbunden ist, wobei im ersten und vierten Drehgeber die Drehbewegung der Welle gegenüber dem Gehäuse wahlweise sperrbar ist.

8. Vorrichtung nach Anspruch 1, dadurch gekennzeichnet, daß die Trägervorrichtung (6) in einem raumfesten Punkt gelenkig verankert und gegebenenfalls mit einem längsverschieblichen Trägerteil (7) ausgestattet ist und daß zusätzlich zu dem zwischen Halteteil (1) und Trägervorrichtung (6) angeordneten Meßsystem (X) ein weiteres Meßsystem (11-12, 14E-15E, 14F-15F) zur Erfassung der Bewegungen des Halteteiles (1) in einem raumfesten Korrdinatensystem vorgesehen ist.

9. Vorrichtung nach Anspruch 8, dadurch gekennzeichnet, daß der längsverschiebliche Trägerteil (7) der Trägervorrichtung (6) vorzugsweise unter Gewichtsentlastung der Höhe nach frei beweglich ist.

10. Vorrichtung nach einem der Ansprüche 8 oder 9, dadurch gekennzeichnet, daß die Trägervorrichtung (6) um ihre Längsachse im Raum drehfest angeordnet ist.

11. Vorrichtung nach Anspruch 8, dadurch gekennzeichnet, daß zwischen dem längsverschieblichen (7) und einem raumfest verankerten Trägerteil (9) ein Längenmeßfühler (11-12) angeordnet ist.

12. Vorrichtung nach Anspruch 8 oder 11, dadurch gekennzeichnet, daß mindestens ein den um eine oder mehrere Achsen erfolgenden Winkelausschlag der Trägervorrichtung (6) messender Meßfühler (14E-15E, 14F-15F) vorgesehen ist.

13. Vorrichtung nach Anspruch 12, dadurch gekennzeichnet, daß die gelenkige Verankerung der Trägervorrichtung (6) im raumfesten Punkt durch eine kinematische Kette bildende Meßfühler (14E-15E, 14F-15F) erfolgt.

**Claims**

1. Device for diagnosing disorders of the motor system of the human body by measuring the position and/or motion of a body part, at least its rotations being measured by means of a measuring device consisting of a holder (1) to be fixedly connected with the body part, a spatially fixed supporting device (6) for the holder (1) and a measuring system (X) consisting of measuring sensors for detecting the rotary motions of the holder relative to the supporting device, characterized in that the holder (1) is connected with the supporting device (6) at a single point by an articulated linkage permitting free motion of the body part, and that the supporting device (6) is so constructed that the movements of the measuring system (X) or of the holder (1) are detectable in a spatially fixed system of coordinates.

2. Device according to claim 1, characterized in that the articulated linkage between holder (1) and supporting device (6) occurs through the measuring system (X) itself which consists preferably of at least three rotary measuring sensors (4A to D, 5A to D) forming a kinematic chain whose axes of rotation (A, B, C ; B, C, D) define a three-dimensional axial system.

3. Device according to claim 2, characterized in that the rotary measuring sensors (4A to D, 5A to D) are rotary transmitters known *per se*, for example rotary potentiometers.

4. Device according to one of claim 2 or 3, characterized in that the rotary measuring sensors (4A to D, 5A to D) are arranged in series, the members of the rotary measuring sensors movable relative to one another being arranged in such a manner that one member (4A) of the first rotary measuring sensor is connected with the holder (1), the other member (5A) of the first rotary measuring sensor is connected with a member (e. g. 5B) of the second rotary measuring sensor, and that a member (4C, 4D) of the last rotary measuring sensor is connected with an element (7) of the supporting device (6).

5. Device according to claim 4 wherein, especially for measuring the position and movement of the head, three rotary transmitters, e. g. rotary potentiometers, are provided, characterized in that the housing (4A) of the first rotary transmitter is connected with the body part, e. g. by means of a helmet (1) with the head, the shafts (5A) of the first rotary transmitter and the housing (4B) of the second rotary transmitter with the shaft (5C) of the third rotary transmitter whose housing (4C) is attached to the spatially fixed supporting device (6).

6. Device according to claim 4 wherein, especially for measuring the position and movement of the head, three rotary transmitters, e. g. rotary potentiometers, are provided, characterized in that the shaft of the first rotary transmitter is connected with the body part, e. g. by means of a helmet with the head, the housing of the first rotary transmitter with the shaft of the second rotary transmitter and the housing thereof with the shaft of the third rotary transmitter whose housing is attached to a part of the spatially fixed suspension device.

7. Device according to claim 5, characterized in that between the third rotary transmitter and the supporting device (6) there is disposed a fourth rotary transmitter (4D, 5D) whose shaft (5D) is connected with the shaft (5C) of the third rotary transmitter and the housing (4D) thereof with an element (7) of the supporting device (6), the rotary motion of the shaft relative to the housing being optionally arrestable in the first and the fourth rotary transmitter.

8. Device according to claim 1, characterized in that the supporting device (6) is articulatedly anchored at a spatially fixed point and is, possibly, provided with a longitudinally displaceable support element (7) and that in addition to the measuring system (X) arranged between the holder (1) and the supporting device (6) a further measuring system (11-12, 14E-15E, 14F-15F) is provided for detecting the movements of the holder (1) in a spatially fixed system of coordinates.

9. Device according to claim 8, characterized in that the longitudinally displaceable support element (7) of the supporting device (6) is vertically freely movable, preferably in a weight-relieving mode.

10. Device according to claim 8 or 9, characterized in that the supporting device (6) is disposed in space non-rotatably about its longitudinal axis.

11. Device according to claim 8, characterized in that a longitudinal measuring sensor (11-12) is arranged between the longitudinal displaceable (7) and a spatially fixed support element (9).

12. Device according to claim 8 or 11, characterized in that at least one measuring sensor (14E-15E, 14F-15F) measuring the angular deflection of the supporting device (6) occurring about one or more axes is provided.

13. Device according to claim 12, characterized in that the articulated anchorage of the supporting device (6) at the spatially fixed point occurs through the measuring sensors (14E-15E, 14F-15F) forming a kinematic chain.

**Revendications**

1. Dispositif pour diagnostiquer des troubles de la motricité du corps humain grâce à une mesure de la position et/ou du déplacement d'une partie du corps, auquel cas on mesure au moins ses rotations à l'aide d'un dispositif de mesure qui est constitué par une partie de maintien (1) devant être reliée fermement à la partie du corps, par un dispositif de support (6) monté fixe dans l'espace et prévu pour la partie de maintien (1) et par un système de mesure (X) formé de capteurs de mesure servant à détecter les mouvements de rotation de la partie de maintien par rapport au dispositif de support, caractérisé en ce que la partie de maintien (1) est reliée au dispositif de support (6) en un point unique par l'intermédiaire d'une liaison articulée autorisant un déplacement libre de la partie du corps, et que le dispositif de support (6) est agencé de telle sorte que des déplacements du système de mesure (X) ou de la partie de maintien (1) peuvent être détectés dans un système de coordonnées fixe dans l'espace.

2. Dispositif selon la revendication 1, caractérisé en ce que la liaison articulée entre la partie de maintien (1) et le dispositif de support (6) est réalisée par le système de mesure (X) lui-même, qui est constitué de préférence par au moins trois capteurs de mesure de rotation (4A à D, 5A à D) formant une chaîne cinématique et dont les axes de rotation (A, B, C ; B, C, D) définissent un système tridimensionnel d'axes.

3. Dispositif selon la revendication 2, caractérisé en ce que les capteurs de mesure de rotation (4A à D, 5A à D) sont de façon connue en soi des capteurs rotatifs, par exemple des potentiomètres rotatifs.

4. Dispositif selon la revendication 2 ou 3, caractérisé en ce que les capteurs de mesure de rotation (4A à D, 5A à D) sont disposés en série, auquel cas les organes, mobiles les uns par rapport aux autres, des capteurs de mesure de rotation sont disposés de telle sorte qu'un organe (4A) du premier capteur de mesure de rotation est relié à la partie de maintien (1), que l'autre organe (5A) du premier capteur de mesure de rotation est relié à un organe (par exemple 5B) du second capteur de mesure de rotation, et qu'un organe (4C, 4D) du dernier capteur de mesure de rotation est relié à une partie (7) du dispositif de support (6).

5. Dispositif selon la revendication 4, dans lequel il est prévu trois capteurs rotatifs, par exemple trois potentiomètres rotatifs, notamment pour la mesure de la position et du déplacement de la tête, caractérisé en ce que le boîtier (4A) du premier capteur rotatif est relié à la partie du corps, par exemple au moyen d'un casque (1) à la tête, que l'arbre (5A) du premier capteur rotatif est relié à l'arbre (5B) du second capteur rotatif et que le boîtier (4B) du second capteur rotatif est relié à l'arbre (5C) du troisième capteur rotatif, dont le boîtier (4C) est fixé à une partie (7) du dispositif de support (6) monté fixe dans l'espace.

6. Dispositif selon la revendication 4, dans lequel il est prévu trois capteurs rotatifs, par exemple des potentiomètres rotatifs notamment pour la mesure de la position et du déplacement de la tête, caractérisé en ce que l'arbre du premier capteur rotatif est relié à la partie du corps, par exemple au moyen d'un casque à la tête, que le boîtier du premier capteur rotatif est relié à l'arbre du second capteur rotatif et que le boîtier de ce dernier est relié à l'arbre du troisième capteur rotatif, dont le boîtier est fixé sur une partie du dispositif de support monté fixe dans l'espace.

7. Dispositif selon la revendication 5, caractérisé en ce qu'entre le troisième capteur rotatif et le dispositif de support (6) se trouve disposé un quatrième capteur rotatif (4D, 5D), dont l'arbre (5D) est relié à l'arbre (5C) du troisième capteur rotatif et dont le boîtier (4D) est relié à une partie (7) du dispositif de support (6), auquel cas dans les premier et quatrième capteurs rotatifs le mouvement de rotation de l'arbre par rapport au boîtier peut être bloqué à volonté.

8. Dispositif selon la revendication 1, caractérisé en ce que le dispositif de support (6) est ancré de façon articulée au niveau d'un point fixe dans l'espace et est équipé éventuellement d'une partie de support (7) déplaçable longitudinalement et qu'en plus du système de mesure (X) disposé entre la partie de maintien (1) et le dispositif de support (6), il est prévu un autre système de mesure (11-12, 14E-15E, 14F-15F) pour détecter les déplacements de la partie de maintien (1) dans un système de coordonnées fixe dans l'espace.

9. Dispositif selon la revendication 8, caractérisé en ce que la partie de support (7) déplaçable longitudinalement du dispositif de support (6) est librement mobile en hauteur, de préférence moyennant une décharge de poids.

10. Dispositif selon l'une des revendications 8 ou 9, caractérisé en ce que le dispositif de support (6) est monté avec blocage en rotation dans l'espace autour de son axe longitudinal.

11. Dispositif suivant la revendication 8, caractérisé en ce qu'un capteur de mesure de longueur (11-12) est monté entre la partie de support (7) déplaçable longitudinalement et une partie de support (9) ancrée de façon fixe dans l'espace.

12. Dispositif selon la revendication 8 ou 11, caractérisé en ce qu'il est prévu au moins un capteur de mesure (14E-15E, 14F-15F) mesurant la déviation angulaire du dispositif de support (6), qui s'effectue autour d'un ou de plusieurs axes.

13. Dispositif selon la revendication 12, caractérisé en ce que l'ancrage par articulation du dispositif de support (6) en un point fixe dans l'espace est réalisé au moyen de capteurs de mesure (14E-15E, 14F-15F) formant une chaîne cinématique.

Fig. 1

Fig. 2

5C
5B
5A
1

C
4C
B
4B
4A
10 A

Fig. 3

9
7
8    8

0 092 541

Fig. 4

Fig. 5